# EUROPÄISCHE PATENTANMELDUNG

(11) **EP 3 308 832 A1**
(43) Veröffentlichungstag der Anmeldung: **18.04.2018**
(21) Anmeldenummer: 16193885.7
(22) Anmeldetag: 14.10.2016
(51) Int. Cl.: A61N 1/375, A61N 1/378, A61N 1/05

(54) **ELEKTRISCHES IMPLANTAT ODER IMPLANTATSYSTEM**

(71) Anmelder: BIOTRONIK SE & Co. KG, 12359 Berlin (DE)
(72) Erfinder: Dörr, Thomas, 12437 Berlin (DE)
(74) Vertreter: Randoll, Sören

(57) **Zusammenfassung**

Die Erfindung betrifft ein elektrisches Implantat oder Implantatsystem, das eine Energieversorgungseinheit aufweist oder mit einer solchen Energieversorgungseinheit verbunden ist und das mindestens eine Fixiervorrichtung zum dauerhaften Fixieren des elektrischen Implantats oder Implantatsystems am Körpergewebe aufweist oder mit einer solchen Fixiervorrichtung verbunden ist. Es wird ein aktives elektrisches Implantat oder Implantatsystem vorgeschlagen, das mindestens zwei lösbar mechanisch miteinander verbundene Implantatkomponenten aufweist, von denen eine erste der Implantatkomponenten wenigstens Teile der Fixiervorrichtung aufweist. Eine zweite der Implantatkomponenten weist eine elektromechanische Komponente auf, die infolge von Stromentnahme oder Stromeinspeisung derart anschwellen kann, dass sich eine Schwellung an der Außenkontur der elektromechanischen Komponente an mindestens einer Stelle ergibt. Die elektromechanische Komponente ist derart angeordnet, dass eine Schwellung der elektromechanischen Komponente ein Trennen der mechanischen Verbindung zwischen der ersten und zweiten Implantatkomponente bewirkt.

## Beschreibung

Die Erfindung betrifft ein elektrisches Implantat oder Implantatsystem, das eine Energieversorgungseinheit aufweist oder mit einer solchen Energieversorgungseinheit verbunden ist und das mindestens eine Fixiervorrichtung zum dauerhaften Fixieren des elektrischen Implantats oder Implantatsystems am Körpergewebe aufweist oder mit einer solchen Fixiervorrichtung verbunden ist.

Derartige elektrische Implantate oder Implantatsysteme können beispielsweise vollständig in eine Herzkammer implantierbare Herzschrittmacher sein, bei denen die Stimulationselektrodenpole direkt am Gehäuse des Herzschrittmachers angeordnet sind, sodass keine Elektrodenleitungen erforderlich sind. Derartige Herzschrittmacher sind als *implantable leadless pacemaker* (ILP) bekannt. Um eine zuverlässige Stimulation der jeweiligen Herzkammern mittels des ILP sicherzustellen, ist es nötig, einen solchen Herzstimulator als aktives elektrisches Implantat in der jeweiligen Herzkammer zu fixieren. Beispielsweise kann sich eine Fixiervorrichtung des elektrischen Implantats in der Trabekelstruktur des Ventrikels verankern, mit der Folge, dass das Implantat nur noch sehr schwer oder gar nicht zu explantieren ist.

Der Erfindung liegt die Aufgabe zugrunde, einen Beitrag zur Lösung dieses Problems zu leisten.

Erfindungsgemäß wird hierzu ein aktives elektrisches Implantat oder Implantatsystem der eingangs genannten Art vorgeschlagen, das mindestens zwei lösbar mechanisch miteinander verbundene Implantatkomponenten aufweist, von denen eine erste der Implantatkomponenten wenigstens Teile der Fixiervorrichtung aufweist. Eine zweite der Implantatkomponenten weist eine elektromechanische Komponente auf, die infolge von Stromentnahme oder Stromeinspeisung derart anschwellen kann, dass sich eine Schwellung an der Außenkontur der elektromechanischen Komponente an mindestens einer Stelle ergibt. Die elektromechanische Komponente ist derart angeordnet, dass eine Schwellung der elektromechanischen Komponente ein Trennen der mechanischen Verbindung zwischen der ersten und zweiten Implantatkomponente bewirkt.

Hieraus ergibt sich der Vorteil, dass wenigstens die zweite Implantatkomponente nach einem Trennen von der ersten Implantatkomponente vergleichsweise leicht explantierbar ist, während die erste Implantatkomponente gegebenenfalls am Implantationsort verbleiben kann. Ein weiterer Vorteil ist, dass sich das Trennen der ersten Implantatkomponente von der zweiten Implantatkomponente elektrisch steuern lässt. Damit kann ein derartiges Trennen beispielsweise auch fernausgelöst werden, indem entsprechende Steuersignale zu dem aktiven elektrischen Implantat oder Implantatsystem übermittelt werden.

Die elektromechanische Komponente weist vorzugsweise einen elektrischen Energiespeicher auf, der seinerseits beispielsweise eine Batterie und/oder einen Kondensator aufweist. Batterien haben häufig die Eigenschaft, anzuschwellen, wenn Sie tiefenentladen werden, sodass die elektromechanische Komponente so gestaltet werden kann, dass ein Tiefenentladen der Batterie ein gezieltes Schwellen an der Außenkontur der elektromechanischen Komponente zur Folge hat. Kondensatoren wiederum können schwellen, wenn sie über eine bekannte Höchstspannung hinaus geladen werden. Entsprechend kann auch der Kondensator der elektromechanischen Komponente so angeordnet sein, dass sich eine Schwellung an der Außenkontur der elektromechanischen Komponente ergibt. Tatsächlich können sogar eine einzelne Batterie oder ein einzelner Kondensator die elektromechanische Komponente darstellen.

Falls die elektromechanische Komponente eine Batterie aufweist, ist es bevorzugt, wenn das aktive elektrische Implantat oder Implantatsystem dazu konfiguriert ist, die Batterie zum Auslösen der Schwellung der elektromechanischen Komponente tiefenzuentladen.

Falls die elektromechanische Komponente ein Kondensator ist, oder einen solchen aufweist, ist das aktive elektrische Implantat oder Implantatsystem vorzugsweise dazu konfiguriert, den Kondensator zum Auslösen der Schwellung der elektromechanischen Komponente über die Nennspannung des Kondensators hinaus aufzuladen.

Falls die elektromechanische Komponente ein Elektrolytkondensator ist oder einen solchen aufweist, ist das aktive elektrische Implantat oder Implantatsystem vorzugsweise dazu ausgebildet, den Elektrolytkondensator zum Auslösen der Schwellung der elektromechanischen Komponente entgegen seiner Polarität zu laden.

Vorzugsweise ist die elektromechanische Komponente ein verkapseltes Bauelement, das bei Anlegen einer Spannung oder Stromeinprägung eine Schwellung verursacht, indem eine Gasbildung infolge einer Elektrolyse in dem verkapselten Bauelement stattfindet. Weiterhin ist es bevorzugt, wenn das elektrische Implantat oder Implantatsystem eine Telemetrieeinheit aufweist, und dazu ausgebildet ist, eine Schwellung der elektromechanischen Komponente nach Empfang eines entsprechenden Telemetriesignals auszulösen.

Besonders bevorzugt ist es, wenn die elektromechanische Komponente Teil der Energieversorgungseinheit des elektrischen Implantats oder Implantatsystems ist und eine Batterie aufweist, wobei die elektromechanische Komponente derart dimensioniert ist, dass sie bei vollständiger Batterieerschöpfung der Energieversorgungseinheit ein Trennen der mechanischen Verbindung zwischen der ersten und der zweiten Implantatkomponente bewirkt. Diese Ausführungsvariante bringt den Vorteil mit sich, dass sich die erste und die zweite Implantatkomponente automatisch voneinander trennen, wenn die Batterie der Energieversorgungseinheit erschöpft ist und infolgedessen anschwillt.

Vorzugsweise ist daher die Batterie der Energieversorgungseinheit derart angeordnet und dimensioniert, dass sie bei vollständiger Batterieerschöpfung soweit anschwillt, dass sie ein Trennen der mechanischen Verbindung zwischen der ersten und der zweiten Implantatkomponente bewirkt.

In einer vorteilhaften Ausführungsvariante ist die mechanische Verbindung zwischen der ersten und der zweiten Implantatkomponente eine Rastverbindung und die bei Stromentnahme oder Stromeinspeisung anschwellende Außenkontur der elektromechanischen Komponente ist im miteinander verrasteten Zustand der beiden Implantatkomponenten an der Außenkontur der ersten Implantatkomponente derart benachbart, dass ein Anschwellen der Außenkontur eine derartige Kraft zwischen der Außenkontur der ersten Implantatkomponente und der Außenkontur der zweiten Implantatkomponente bewirkt, dass sich die Rastverbindung löst.

Das aktive elektrische Implantat oder Implantatsystem ist vorzugsweise ein implantierbarer elektrodenleitungsloser Herzschrittmacher, also ein *implantable leadless pacemaker* (ILP). Ebenfalls vorteilhaft ist es, wenn das aktive elektrische Implantat oder Implantatsystem ein implantierbarer Sensor ist.

In einer weiteren vorteilhaften Ausführungsvariante ist das elektrische Implantat oder Implantatsystem eine Sensor- oder Elektrodenleitung.

Die zum Fixieren oder Verankern am Implantationsort vorgesehene erste Implantatkomponente ist mit Gewebeankern als Fixiervorrichtung versehen.
Die Erfindung soll nun anhand von Ausführungsbeispielen mit Bezug auf die Figuren näher erläutert werden. Von den Figuren zeigt:
- Fig. 1a: eine schematische Darstellung eines erfindungsgemäßen Implantats mit zwei Implantatkomponenten in deren mechanisch verbundenen Zustand
- Fig. 1b: das Implantat aus Fig. 1a mit mechanisch voneinander gelösten Implantatkomponenten
- Fig. 2a: eine schematische Darstellung eines Implantats mit zwei mechanisch miteinander verbundenen Implantatkomponenten einschließlich einer Darstellung einiger Teilkomponenten der zweiten Implantatkomponente
- Fig. 2b: das Implantat aus Fig. 2a mit mechanisch voneinander getrennten Implantatkomponenten
- Fig. 3a: eine alternative Darstellung eines Implantats mit zwei mechanisch miteinander verbundenen Implantatkomponenten und
- Fig. 3b: das Implantat aus Fig. 3a mit mechanisch voneinander getrennten Implantatkomponenten

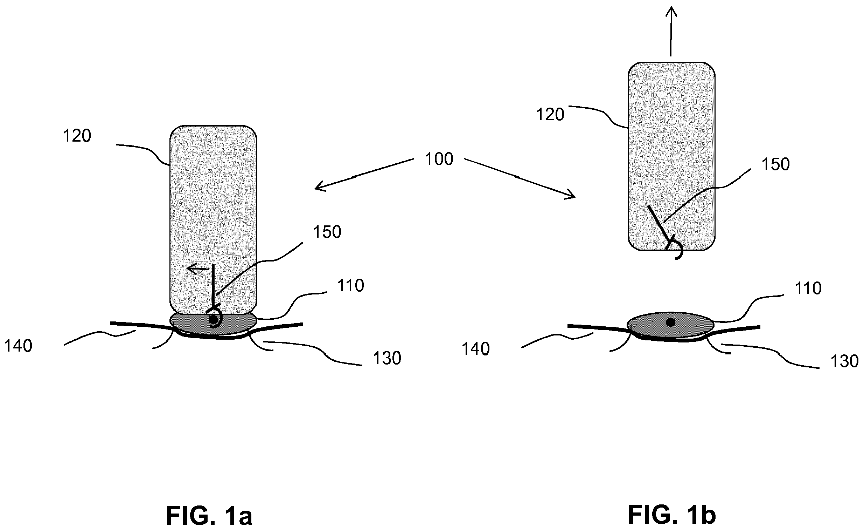

Fig. 1 zeigt einen implantierbaren elektrodenleitungslosen Herzschrittmacher 100 als Beispiel für ein aktives elektrisches Implantat oder Implantatsystem. Der dieser Erfindung zugrunde liegende Gedanke ist jedoch bei anderen Implantaten oder Implantatsystemen anwendbar einschließlich solcher Implantatsysteme, die Elektrodenleitungen aufweisen.

Der in Figur 1 abgebildete Herzschrittmacher 100 ist teilexplantierbar und besitzt einen ersten Implantatteil 110, der als erste Implantatkomponente eine Fixiereinrichtung in Form von Nitinolankern 130 aufweist. Mechanisch mit dem ersten Implantatteil 110 ist ein zweiter, aktiver Implantatteil 120 lösbar verbunden, der in üblicherweise eine Batterie und Steuerelektronik umfasst (in Fig. 1 nicht dargestellt). Die Nitinolanker 130 des ersten Implantatteils 110 dienen als Fixiervorrichtung zur Verankerung des Implantats 100 mit beispielsweise Herzgewebe. Nach der Implantation des Implantats 100 verwachsen die Nitinolanker 130 typischerweise fest mit Myokardgewebe 140. Typischerweise wachsen derartige Anker derart in das Gewebe ein, dass sie nach einigen Wochen nur noch chirurgisch entfernt werden können. Der erste Implantatteil 110 kann daher praktisch nicht mehr aus dem Gewebe entfernt werden.

Daher ist die mechanische Verbindung 150 zwischen dem ersten Implantatteil 110 und dem zweiten, aktiven Implantatteil 120 lösbar und in Form einer Verriegelungsvorrichtung ausgeführt. Diese erlaubt es, den zweiten, aktiven Implantatteil nach Lösen der Verriegelungsvorrichtung 150 zu explantieren falls beispielsweise dessen Batterie erschöpft ist. Das Lösen der Verriegelungsvorrichtung 150 kann dabei durch eine entsprechende Programmierung des Implantats 100 erfolgen. Anschließend kann der zweite, aktive Implantatteil 120 explantiert werden, während der erste Implantatteil 110 mit der Fixiervorrichtung und insbesondere den Ankern 130 im Herzen verbleiben.

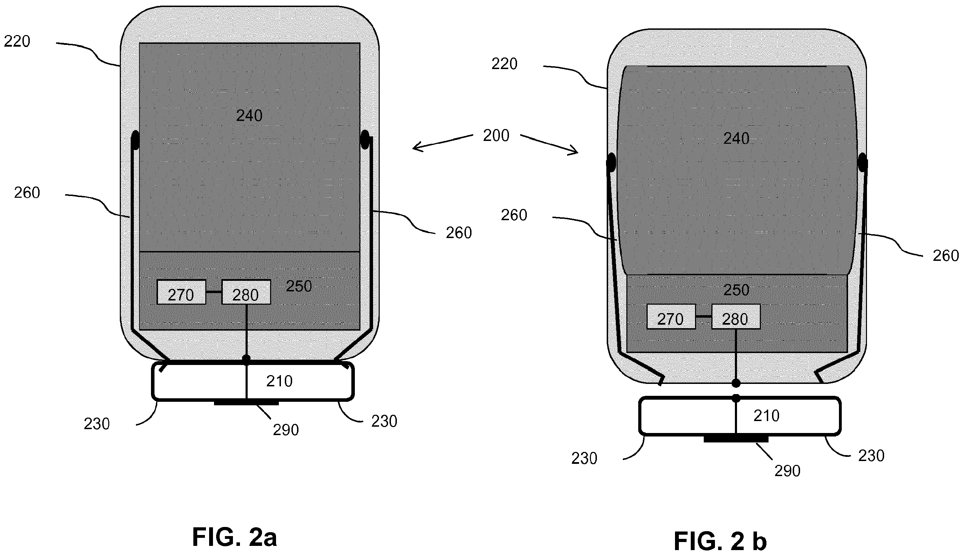

Figur 2 zeigt eine Variante des Implantats 200 in Form eines Herzschrittmachers in etwas detaillierterer Darstellung. Zu erkennen ist wieder, dass das Implantat 200 wiederrum aus einem ersten Implantatteil 210 und einem zweiten, aktiven Implantatteil 220 zusammengesetzt ist. Das erste Implantatteil 210 weist wiederrum eine Fixiervorrichtung auf, die unter anderem aus beispielsweise Nitinol gefertigte Anker 230 umfasst, die nach der Implantation mit dem Myokard verwachsen können. Außerdem ist an dem ersten Implantatteil 210 eine Stimulationselektrode 290 angeordnet, die nach Implantation im elektrischen Kontakt mit dem Myokard steht.

Die zweite, aktive Implantatkomponente 220 enthält eine Energiequelle in Form einer Batterie 240 und eine mit der Batterie elektrisch verbundene Steuer- und Therapieelektronik 250. Letzte umfasst unter anderem eine Telemetrieeinheit 270 und eine Stimulationseinheit 280.

Der erste Implantatteil 210 und der zweite Implantatteil 220 sind in dem dargestellten Ausführungsbeispiel mittels zweier Verriegelungshebel 260 mechanisch miteinander verbunden. Die Verriegelungshebel 260 sind so angeordnet, dass sie die Verriegelung zwischen dem ersten Implantatteil 210 und dem zweiten Implantatteil 220 freigeben, wenn die Batterie 240 anschwillt und eine anschwellende Außenkontur der Batterie 240 auf entsprechende Arme der Verriegelungshebel 260 drückt.

Ein Anschwellen der Batterie 240 erfolgt beispielsweise dann, wenn diese vollständig an ihrem Lebensende entladen ist. Das Lebensende der Batterie 240 führt auf diese Weise automatisch zu einer Lösung mit der mechanischen Verbindung zwischen dem ersten Implantatteil 210 und dem zweiten Implantatteil 220.

Alternativ oder zusätzlich kann die Steuer- und Therapieelektronik 250 auch so ausgebildet sein, dass sie auf ein entsprechendes Telemetriesignal hin ein Entladen der Batterie 240 und damit deren Anschwellen auslöst, mit der Folge, dass die mechanische Verbindung zwischen der ersten Implantatkomponente 210 und der zweiten Implantatkomponente 220 getrennt wird. Hierzu weist die Steuer- und Therapieelektronik 250 die Telemetrieeinheit 270 auf.

Die Batterie 240 ist also als elektromechanische Komponente ausgebildet, die so ausgelegt ist, dass sie an vorgesehenen Stellen ihrer Außenkontur anschwillt, wenn sie tiefenentladen wird. Alternative elektromechanische Komponenten können z.B. Kondensatoren zusätzlich oder alternativ zu der Batterie enthalten.

Wie Figur 2 ebenfalls zu entnehmen ist, weist die erste, mit dem Myokard verankerte Implantatkomponente 210 eine Stimulationselektrode 270 auf, die im mechanisch verbundenen Zustand der beiden Implantatkomponenten 210 und 220 elektrisch mit der Stimulationseinheit 280 verbunden ist. Auch diese elektrische Verbindung zwischen der Stimulationselektrode 290 und der Stimulationseinheit 280 löst sich nach dem mechanischen Trennen der ersten Implantatkomponente 210 von der zweiten Implantatkomponente 220. Es sei angemerkt, dass die Elektrode 290 auch eine Abfühlelektrode sein kann und entsprechend anstelle der Stimulationseinheit 280 auch eine entsprechende Sensingeinheit vorgesehen sein kann. Es können Kombinationen dieser Einheiten vorgesehen sein. Darüberhinaus kann das Implantat 200 auch weitere Stimulations- oder Sensingelektroden aufweisen.

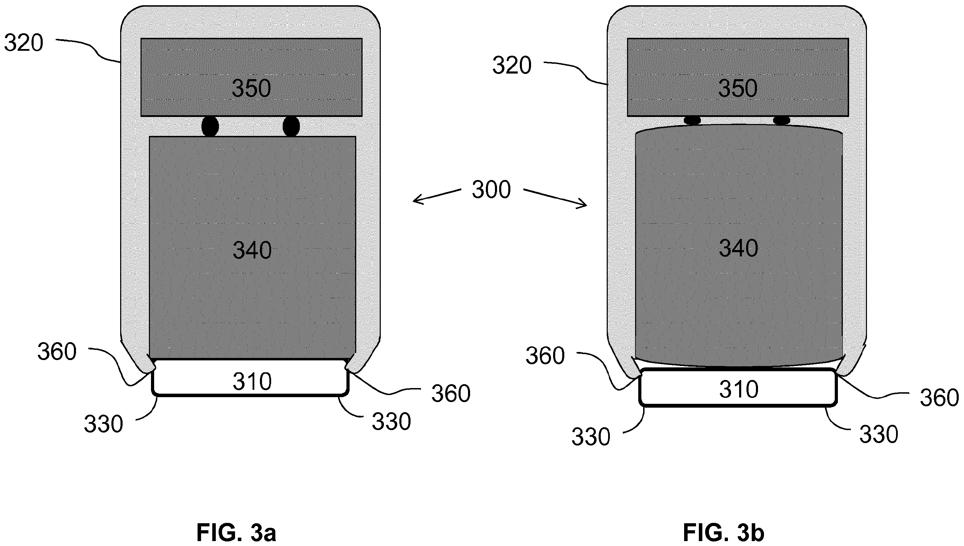

Figur 3 zeigt eine alternative Ausführungsform eines Implantats 300 (elektrodenleitungsloser Herzschrittmacher) mit einem ersten Implantatteil 310, das über Gewebeanker 330 zur Verankerung mit Myokard verfügt und einem zweiten Implantatteil 320, der als aktives Implantatteil eine Batterie 340 sowie elektronische Komponenten 350 aufweist. Die Verriegelung zwischen dem ersten Implantatteil 310 und dem zweiten Implantatteil 320 ist in diesem Falle über eine Rastverbindung 360 realisiert, bei der Rastvorsprünge an dem zweiten Implantatteil 320 in entsprechende Rastvertiefungen an dem ersten Implantatteil 310 eingreifen.

Wenn die Batterie 340 beispielsweise zum Ende ihres Lebenszykluss oder infolge einer gezielten Entladung nach Empfang eines entsprechenden Telemetriesignals anschwillt, drückt ihre anschwellende Außenkontur auf eine dieser gegenüberliegende Außenkontur der ersten Implantatkomponente 310. Die Verriegelung mittels Rastverbindung zwischen erster Implantatkomponente 310 und zweiter Implantatkomponente 320 ist dabei so ausgelegt, dass das Anschwellen der Batterie 340 ein Lösen der Rastverbindung bewirkt, sodass anschließend der erste Implantatteil 310 und der zweite Implantatteil 320 mechanisch voneinander getrennt sind.

### Bezugszeichenliste

- 100, 200, 300: Implantat (Herzschrittmacher)
- 110, 310, 310: erstes Implantatteil
- 120, 220, 320: zweites Implantatteil
- 130: Nitinolanker
- 140: Myokardgewebe
- 150: mechanische Verbindung (Verriegelungsvorrichtung)
- 230: Anker
- 240, 340: Elektromechanische Komponente (Batterie)
- 250: Steuer-/Therapieelektronik
- 260: Verriegelungshebel
- 270: Telemetrieeinheit
- 280: Stimulationseinheit
- 290: Stimulationselektrode
- 350: elektronische Komponente
- 360: Rastverbindung

## Patentansprüche

1. Aktives elektrisches Implantat (100, 200, 300) oder Implantatsystem,
das eine Energieversorgungseinheit aufweist oder mit einer solchen verbunden ist, und das mindestens eine Fixiervorrichtung zur dauerhaften Fixierung an Körpergewebe aufweist oder mit einer solchen verbunden ist,
wobei das aktive elektrische Implantat oder Implantatsystem mindestens zwei lösbar mechanisch miteinander verbundene Implantatkomponenten aufweist,
von denen eine erste der Implantatkomponenten wenigstens Teile der Fixiervorrichtung aufweist und
von denen eine zweite der Implantatkomponenten eine derartige elektromechanische Komponente (240, 340) aufweist, welche infolge von Stromentnahme oder Stromeinspeisung anschwillt, so dass sich eine Schwellung an der Außenkontur der elektromechanischen Komponente an mindestens einer Stelle ergibt, wobei die elektromechanische Komponente (240, 340) derart angeordnet ist, dass eine derartige Schwellung der elektromechanischen Komponente (240, 340) ein Trennen der mechanischen Verbindung zwischen der ersten und der zweiten Implantatkomponente bewirkt.

2. Aktives elektrisches Implantat (100, 200, 300) oder Implantatsystem nach Anspruch 1, **dadurch gekennzeichnet, dass** die elektromechanische Komponente (240, 340) einen elektrischen Energiespeicher aufweist.

3. Aktives elektrisches Implantat (100, 200, 300) oder Implantatsystem nach Anspruch 2, **dadurch gekennzeichnet, dass** der elektrische Energiespeicher eine Batterie (240, 340) und/oder einen Kondensator aufweist.

4. Aktives elektrisches Implantat (100, 200, 300) oder Implantatsystem nach Anspruch 3, **dadurch gekennzeichnet, dass** die elektromechanische Komponente eine Batterie (240, 340) ist oder aufweist und dass das aktive elektrische Implantat oder Implantatsystem dazu konfiguriert ist, die Batterie zum Auslösen der Schwellung der elektromechanischen Komponente tiefenzuentladen.

5. Aktives elektrisches Implantat (100, 200, 300) oder Implantatsystem nach Anspruch 3, **dadurch gekennzeichnet, dass** die elektromechanische Komponente (240, 340) ein Kondensator ist oder einen solchen aufweist, und dass das aktive elektrische Implantat (100, 200, 300) oder Implantatsystem dazu konfiguriert ist, den Kondensator zum Auslösen der Schwellung der elektromechanischen Komponente über dessen Nennspannung aufzuladen.

6. Aktives elektrisches Implantat (100, 200, 300) oder Implantatsystem nach Anspruch 3, **dadurch gekennzeichnet, dass** die elektromechanische Komponente (240, 340) ein Elektrolytkondensator ist oder einen solchen aufweist, und dass das aktive elektrische Implantat oder Implantatsystem dazu konfiguriert ist, den Elektrolytkondensator zum Auslösen der Schwellung der elektromechanischen Komponente entgegen seiner Polarität zu laden.

7. Aktives elektrisches Implantat (100, 200, 300) oder Implantatsystem nach wenigstens einem der Ansprüche 1 bis 6, **dadurch gekennzeichnet, dass** die elektromechanische Komponente (240, 340) ein verkapseltes Bauelement ist, das bei Anlegen einer Spannung oder Stromeinprägung eine Schwellung verursacht, indem eine Gasbildung in Folge einer Elektrolyse im Bauelement stattfindet.

8. Aktives elektrisches Implantat (100, 200, 300) oder Implantatsystem nach wenigstens einem der Ansprüche 1 bis 7, **dadurch gekennzeichnet, dass** das aktive elektrische Implantat (100, 200, 300) oder Implantatsystem eine Telemetrieeinheit (270) aufweist und ausgebildet ist, eine Schwellung der elektromechanischen Komponente (240, 340) nach Empfang eines entsprechenden Telemetriesignals auszulösen.

9. Aktives elektrisches Implantat (100, 200, 300) oder Implantatsystem nach wenigstens einem der Ansprüche 1 bis 8, **dadurch gekennzeichnet, dass** die elektromechanische Komponente (240, 340) Teil der Energieversorgungseinheit ist und eine Batterie (240, 340) aufweist, wobei die elektromechanische Komponente (240, 340) derart dimensioniert ist, dass sie bei vollständiger Batterieerschöpfung (EOS) der Energieversorgungseinheit ein Trennen der mechanischen Verbindung (150, 260, 360) zwischen der ersten und der zweiten Implantatkomponente bewirkt.

10. Aktives elektrisches Implantat (100, 200, 300) oder Implantatsystem nach Anspruch 9, **dadurch gekennzeichnet, dass** die Batterie (240, 340) der Energieversorgungseinheit derart angeordnet und dimensioniert ist, dass sie bei vollständiger Batterieerschöpfung (EOS) soweit anschwillt, dass sie ein Trennen der mechanischen Verbindung (150, 260, 360) zwischen der ersten und der zweiten Implantatkomponente bewirkt.

11. Aktives elektrisches Implantat (100, 200, 300) oder Implantatsystem nach wenigstens einem der Ansprüche 1 bis 10, **dadurch gekennzeichnet, dass** die erste und die zweite Implantatkomponente über eine Rastverbindung (360) mechanisch miteinander verbunden sind und dass die bei Stromentnahme oder Stromeinspeisung anschwellende Außenkontor der elektromechanischen Komponente (260, 360) im miteinander verrasteten Zustand der beiden Implantatkomponenten an einer Außenkontur der ersten Implantatkomponente anliegt.

12. Aktives elektrisches Implantat (100, 200, 300) oder Implantatsystem nach wenigstens einem der Ansprüche 1 bis 11, **dadurch gekennzeichnet, dass** das Implantat ein implantierbarer elektrodenleitungsloser Herzschrittmacher (100, 200, 300) (implantable leadless pacemaker, iLP) ist.

13. Aktives elektrisches Implantat (100, 200, 300) oder Implantatsystem nach wenigstens einem der Ansprüche 1 bis 11, **dadurch gekennzeichnet, dass** das Implantat ein implantierbarer Sensor ist.

14. Aktives elektrisches Implantat (100, 200, 300) oder Implantatsystem nach wenigstens einem der Ansprüche 1 bis 11, **dadurch gekennzeichnet, dass** das Implantat eine Sensor- oder Elektrodenleitung ist.
